Europäisches Patentamt

European Patent Office

Office européen des brevets

(11) Publication number: **0 119 528 B1**

# EUROPEAN PATENT SPECIFICATION

(45) Date of publication of patent specification: **13.05.92** (51) Int. Cl.⁵: **C12P 21/08**, C12N 15/00, C12N 5/00, G01N 33/574

(21) Application number: **84102265.0**

(22) Date of filing: **02.03.84**

(54) Monoclonal antibodies to human renal cancer antigens and method.

(30) Priority: **11.03.83 US 474224**

(43) Date of publication of application:
**26.09.84 Bulletin 84/39**

(45) Publication of the grant of the patent:
**13.05.92 Bulletin 92/20**

(84) Designated Contracting States:
**DE FR GB**

(56) References cited:
**EP-A- 0 073 953**
**EP-A- 0 143 367**

**PROCEEDINGS OF THE NATIONAL ACADEMY OF SCIENCES OF USA, vol. 78, no. 8, August 1981, pages 5122-5126; R. UEDA et al.: "Cell surface antigens of human renal cancer defined by mouse monoclonal antibodies: Identification of tissue-specific kidney glycoproteins"**

(73) Proprietor: **Sloan-Kettering Institute For Cancer Research**
**1275 York Avenue**
**New York New York 10021(US)**

(72) Inventor: **Bander, Neil H.**
**1385 York Avenue**
**New York New York 10021(US)**
Inventor: **Cordon-Cardo, Carlos**
**430 East 67 Street**
**New York New York 10021(US)**
Inventor: **Finstad, Connie L.**
**504 East 81 Street**
**New York New York 10028(US)**
Inventor: **Whitmore, Willet F., Jr.**
**345 East 68 Street**
**New York New York 10021(US)**
Inventor: **Melamed, Myron R.**
**251 Beverly Road**
**Scarsdale New York 10583(US)**
Inventor: **Oettgen, Herbert F.**
**48 Overlook Drive**
**New Canaan, Conn. 06840(US)**

PROCEEDINGS OF THE 72nd ANNUAL MEET-
ING OF THE AMERICAN ASSOCIATION FOR
CANCER RESEARCH, Washington, D.C., US,
27th-30th April 1981, vol. 22, 1981, abstract
no. 1207; R. UEDA et al.: "Human renal can-
cer cell surface antigens defined by mouse
monoclonal antibodies"

CHEMICAL ABSTRACTS, vol. 100, no. 25, 18th
June 1984, page 419, abstract no. 207626n,
Columbus, Ohio, US; P.W. ANDREWS et al.:
"Two monoclonal antibodies recognizing de-
terminants on human embryonal carcinoma
cells react specifically with the liver isozyme
of human alkaline phosphatase", &
HYBRIDOMA 1984, 3(1), 33-9

Inventor: Old, Lloyd J.
600 West End Avenue
New York New York 10024(US)
Inventor: Fradet, Yves
1583 Place Chavigneau
Sainte-Foy Ouebec, G1X 4C7(CA)
Inventor: Lloyd, Kenneth O.
4525 Henry Hudson Parkway
Bronx New York 10471(US)

(74) Representative: Vossius & Partner
Siebertstrasse 4 P.O. Box 86 07 67
W-8000 München 86(DE)

**Description**

The present invention relates to the generation of monoclonal antibodies and their use in identifying or characterizing human renal cancer antigens. This is a useful diagnostic tool in the detection of renal cancer as well as the study of the nature of renal cancer. Red blood cells, immunofluorescent, radioactive or enzymatic tagging agents can be bound to the highly specific antibodies using normal procedures, as required for indexing methods. Cytotoxic agents can also be bound to the highly specific antibodies to produce so called "magic bullet" type therapeutic agents which selectively destroy the cell with which the specific antibody binds.

In 1975 Köhler and Milstein introduced a procedure for the production of monoclonal antibodies (mAbs) using hybrid cells (hybridomas) which allows the production of almost unlimited quantities of antibodies of precise and reproducible specificity. Conventional antisera, produced by immunizing animals with tumor cells or other antigens, contain a myriad of different antibodies differing in their specificity and properties, whereas hybridomas produce a single antibody with uniform characteristics. The Köhler-Milstein procedure entails the fusion of spleen cells from an immunized animal with an immortal myeloma cell line. From the fused cells (hybridomas), clones are selected that produce an antibody of the desired specificity. Each clone continues to produce only that one antibody. As hybridoma cells can be cultured indefinitely (or stored frozen in liquid nitrogen), a constant supply of antibody is assured.

Antibodies are proteins that have the ability to combine with and recognize other molecules, known as antigens. Monoclonal antibodies are no different from other antibodies except that they are very uniform in their properties and recognize only one antigen or a portion of an antigen known as a determinant.

In the case of cells, the determinant recognized is an antigen on or in the cell which reacts with the antibody. It is through these cell antigens that a particular antibody recognizes, i. e. reacts with, a particular kind of cell. Thus the cell antigens are markers by which the cell is identified.

These antigenic markers may be used to observe the normal process of cell differentiation and to locate abnormalities within a given cell system. The process of differentiation is accompanied by changes in the cell surface antigenic phenotype, and antigens that distinguish cells belonging to distinct differentiation lineages or distinguish cells at different phases in the same differentiation lineage may be observed if the correct antibody is available. Initial recognition of differentiation antigens came about through analysis of surface antigens of T-cell leukemias of the mouse and the description of the TL, Thy-1, and Lyt series of antigens. (Old, Lloyd J., Cancer Research, 41, 361-375, February 1981). The analysis of these T-cell differentiation antigens was greatly simplified by the availability of normal T cells and B cells of mouse and man and is relatively advanced; US-Patents 4,361,549-550; 4,364,932-37 and 4,363,799 concerning mAb's to Human T-cell antigens). Little is known about differentiation antigens displayed on normal and neoplastic cells belonging to other lineages.

This is due to the difficulty of obtaining a ready source of the appropriate normal cell type as well as the vagaries of the art of monoclonal antibodies. The preparation of hybrid cell lines can be successful or not depending on such experimental factors as nature of the inoculant, cell growth conditions, hybridization conditions etc. Thus it is not always possible to predict successful hybridoma preparation of one cell line although success may have been achieved with another cell line.

Progress in defining surface antigens on melanocytes was made possible by the recently discovered technique of culturing melanocytes from normal skin (Eisinger, et. al., Proc. Nat'l. Acad. Sci. USA, 79, 2018 (March 1982)). This method provides a renewable source of proliferating cells for the analysis of melanocyte differentiation antigens.

We recently described our initial analysis of cell surface antigens of human malignant melanoma identified by mouse monoclonal antibodies (Abs) (Dippold et al., Proc. Natl. Acad. Sci. USA 77, 6114-6118 (1980)). This invention relates to a comparable analysis of human renal cancer.

Seventeen monoclonal antibodies derived from fusions with spleen cells of mice immunized with established culture lines of renal cancers identified nine cell surface antigenic systems (Ueda, Ryuzo et al, Proc. Natl. Acad. Sci. USA, 78, 5122, August 1981). Six of the systems gp160, $S_{25}$, gp120r, gp120nr, gp115, and $V_1$ represented new antigens not previously described. The other three systems are related to HLA-A, -B, and -C heavy chain and A and B blood group antigens. The most restricted of the newly described antigens were gp160, $S_{25}$, and gp120r. These determinants are found only on cells of renal origin, both normal and malignant, and represent differentiation antigens of human kidney. In addition to the difference in the molecular weight of two of these antigens, gp160, $S_{25}$, and gp120r can be distinguished on the basis of differential expression on a panel of cultured renal cancers and normal kidney epithelium and fetal kidney cells. Glycoproteins bearing gp120r share a determinant with renal gp120nr (as indicated by sequential precipitations with monoclonal antibodies that detect gp120r and gp120nr), but gp120nr is found on a broader range of cell types, including fibroblasts and cell lines derived from ovarian, bladder, and colon cancers. The two other new systems, gp115 and $V_1$, have characteristics of broadly occurring differentiation antigens but can be distinguished from each other and from gp120nr by differences in molecular weight, heat stability ($V_1$ is a heat-stable determinant), and differential expression on cell types of diverse origin.

These systems can be used to characterize and study the nature of renal cancer. Thus, comparison of the $S_{25}$ and gp160 phenotypes of different renal cancer cell lines and cultures of normal kidney clearly distinguish these two systems.

Dippold et al, Proc. Natl. Acad. Sci. USA 77, 6114 - 6118 (1980), have generated a series of mouse Abs that define 12 new systems of human cell surface antigens. Six of these have been identified as glycoproteins (gp95, gp150, gp160, gp120r, gp120nr, and gp115), three are heat-labile antigens that could not be immunoprecipitated from labeled cell extracts ($S_{25}$, $M_{19}$, and $R_8$), and three are heat-stable antigens, presumably glycolipids ($O_5$, $R_{24}$, and $V_1$). The use of a standard panel of cultured human cells allows ready comparisons of the reactivity of these monoclonal antibodies in direct serological tests and absorption analysis, and each of the antigenic systems has a distinct pattern of distribution on the cell panel, in terms of both qualitative and quantitative expression of antigens. On the basis of their distribution on different cell types, these 12 antigenic systems can be further classified into three groups: (i) those with characteristics of restricted differentiation antigens (e. g., the renal-specific gp160, $S_{25}$, and gp120r antigens and the $R_{24}$ antigen of melanoma and melanocytes), (ii) more broadly represented differentiation antigens (e. g., gp95, gp150, $M_{19}$, pg120nr, and $V_1$, and (iii) antigens expressed by every human cell type tested (e. g., $O_5$ species antigen).

It has also been found that the cell lines derived from stage I renal cancer (confined to the kidney) are gp160$^+$, whereas cell lines from metastatic renal cancers are gp160$^-$. Whether this indicates that cancer cells developing metastatic potential lose gp160 expression, or that gp160$^+$ and gp160$^-$ renal cancers are derived from separate cell lineages is not determined; however, identifying the cell types in normal kidney that express gp160 and other antigens found on renal cancer should give information about the cellular origins of renal cancer.

These serological probes provided by the invention can identify kidney-specific antigens and are of particular interest in the study of kidney structure and function. In addition, some of the more broadly reacting antibodies are useful in studying other tumors - e. g. $V_1$ which distinguishes astrocytomas from melanomas.

The importance of parallel biochemical and serological characterizations of antigens identified by Abs is illustrated by the analysis of gp120r and gp120nr. Five Abs in this series immunoprecipitated a 120,000-dalton component from labeled extracts of SK-RC-7 renal cancer cells. Pre-clearing the extract with one of these Abs (AB $S_6$) removed the 120,000-dalton component identified by Ab $S_{23}$, indicating that the two Abs were reacting with the same molecule. However, the antigenic determinant detected by Ab $S_6$ and Ab $S_{23}$ can be distinguished in M-MHA tests and absorption analysis. Ab $S_{23}$ detected a kidney-specific antigen, whereas Ab $S_6$ reacted with a much broader range of cell types. These results can be explained by postulating two species of gp120 molecules, both carrying the epitope identified by Ab $S_6$ but only one with the epitope identified by Ab $S_{23}$. In agreement with this interpretation, supernatants after clearing with Ab $S_{23}$ still reacted with Ab $S_6$, even though no antigen precipitating with Ab $S_{23}$ remained. The epitope identified by Ab $S_{23}$ is found only on cells of renal origin, and, because of this restricted distribution, it is referred to as gp120r. The more widely distributed epitope has been designated "nr" to indicate its nonrestricted nature. gp120r and gp120nr may be the products of two separate genes or of a single gene whose product is modified in renal cells. Similar, although less striking, discrepancies in the cellular distribution of antigens identified by different monoclonal antibodies immunoprecipitating gp95 or gp150 molecules have also been explained on the basis of different epitopes being recognized (Dippold, et al. Proc. Natl. Sci. USA 77, 6114 - 6118 (1980)).

The present invention relates to a monoclonal antibody and to the corresponding monoclonal antibody-producing hybridoma cell line obtainable by fusing a myeloma derived cell strain and splenocytes derived from BALB/c mice immunized with human normal renal epithelial cells, said monoclonal antibody being capable of recognizing human renal epithelial cancer cells. It especially relates to a monoclonal antibody which recognizes the human renal cell antigen gp140, said antigen gp140 being recognizable by the monoclonal reference antibody F23 (ATCC HB 8231).

The new mAb, F23, is described below. F23 is part of a panel of monoclonal antibodies of renal origin used to detect renal cancer cells and antigens. This panel serves an important diagnostic function. The mAbs therein subset renal tumors and subset the normal nephron. The mAbs define renal antigens on normal cells, presumably differentiation antigens; and define renal antigens present on tumors, thus distinguishing normal and cancerous renal cells.

The monoclonal antibody designated F23 recognizes human renal cells and is produced by the hybridoma cell line deposited with the ATCC under Accession No. HB 8231.

The present invention also relates to a method for differentiating between normal and malignant human renal cells which comprises contacting a human renal sample with a plurality of monoclonal antibodies each of which is different and each of which is produced by a hybridoma cell line formed by fusing a myeloma derived cell strain and splenocytes derived from BALB/c mice immunized with established culture lines from the group of human epithelial renal cancer or normal human renal epithelial cells under suitable conditions so as to form a complex between each antibody and an antigen, detecting the presence of any so formed complex, the absence of any complex indicating normal renal cells and the presence of any complex indicating the presence of malignant human renal cells.

In the above method the plurality of monoclonal antibodies can be selected from the group consisting of $F_{23}$, $M_2$, $S_8$ and $S_{21}$.

The above method may further comprise contacting a human renal sample with a plurality of monoclonal antibodies selected from the group consisting of $M_1$, $S_4$, $S_6$, $S_7$, $S_{22}$, $S_{23}$, $S_{24}$, $S_{25}$, $S_{26}$, $S_{27}$ and $V_1$. It may be used as an assay for the malignant potential of human renal tumors.

Description - Techniques

Tissue Culture. The renal cancer cell lines (Ueda et al J. Exp. Med. 150, 564 - 589 (1979)) and tumor cell lines (Carey, et al Proc. Natl. Acad. Sci. USA 73, 3278 - 3282 (1976)) have been described. Methods for the short-term culture of normal kidney epithelium have also been described (Udea (supra)). Cultures were maintained in Eagle's minimal essential medium supplemented with 2 mM glutamine, 1 % nonessential amino acids, 100 units of penicillin per ml, 1 μg of streptomycin per ml, and 10 % (vol/vol) fetal bovine serum. Cultures were regularly tested for mycoplasma, fungi, and bacteria, and contaminated cultures were discarded.

Serological Procedures. The mouse mixed hemadsorption assay (M-MHA) was performed by the method of Fagraeus et al., Immunology 9, 161 175 (1965), as modified to detect mouse antibody. Serological procedures for direct test and absorption analysis are described in Dippold et al (supra); Ueda et al (supra) and Carey et al (supra).

Immunizations. (BALB/C x C57BL/6)$F_1$ female mice were immunized with established renal cancer cell lines. For the initial immunization, 1 x $10^7$ renal cancer cells were injected subcutaneously without adjuvant. Subsequent immunizations were carried out at intervals of 3 - 4 weeks by intraperitoneal inoculation of 1 x $10^7$ renal cancer cells. Immunized mice were sacrificed 3 days after the last immunization.

Derivation of Mouse Abs. The fusion of immune spleen cells with mouse myeloma MOPC-21 NS/l cells was performed as described (Dippold et als (supra) and Köhler & Milstein, Nature (London) 236, 495 - 497 (1975). Fused cells (5 - 8 x $10^5$) in 1 ml of selective medium containing hypoxanthine, aminopterin, and thymidine were added to wells of tissue culture plates (Costar no. 3524, 24 wells per plate). Hybridoma cultures were subcloned at least three times by limiting dilution on a feeder layer of 1 - 3 x $10^5$ mouse peritoneal macrophages. Culture supernatants were monitored for antibody activity on a panel of cultured cells consisting of two renal cancer cell lines (including the immunizing line), AJ astrocytoma, SK-MEL-33 and -37 melanomas, Me-180 cervix cancer, WI-38 fetal cells, VERO adult and fetal kidney epithelium, and fetal brain cells. Antibody subclass was determined by double diffusion in agar with anti-Ig heavy chain specific reagents (Bionetics, Kensington, M. D.). Cultures of cloned hybridomas were injected subcutaneously into nu/nu mice (Swiss background) and were also stored in liquid nitrogen. Sera from mice with progressively growing tumors were collected, stored at -70ºC, and used for serological and biochemical characterization.

Immunoprecipitation Procedures. Cells were metabolically labeled with ($^3$H)glucosamine in complete Eagle's medium containing 15 μCi of ($^3$H)glucosamine (New England Nuclear; 30 - 60 Ci/mmol; 1 Ci = 3.7 x $10^{10}$ becquerels) per ml for 48 hr at 37ºC; the labeled cells were extracted with 0.5 % Nonidet P-40 (NP40) in Tris buffer as described (Ogata et al. Proc. Natl. Acad. Sci. USA 78, 770 - 774 (1981)) except that the 3M KCl treatment was omitted.

5

EP 0 119 528 B1

Immunoprecipitation was carried out by mixing a portion of the cell extract (1 x $10^5$ cpm) with 2 $\mu$l of mouse serum and 20 $\mu$l of rabbit anti-mouse Ig serum (Cappel Laboratories, Cochranville, PA) in Tris buffer). Immune complexes were isolated by using Staphylococcus aureus and analyzed by NaDodSO$_4$/polyacrylamide gel electrophoresis as described (Dippold et al (supra)). ($^{35}$S) Methionine-labeled samples were immunoprecipitated in a similar manner, except that Sepharose-rabbit F (ab')$_2$ anti-mouse Ig was used for isolating the complexes. To determine the pI of the antigens, immunoprecipitates were examined by two-dimensional electrophoresis by the O-Farrell procedure (O'Farrell, P. H. Biol. Chem. 250, 4007 - 4021 (1975)) modified as described (Ogata, et al (supra)). From the five fusions of NS-1 myeloma with three different renal cancer cell lines, 17 antibody-producing clones were selected for detailed analysis (Table 1). The serological specificity of these antibodies was tested on a panel of 47 established cell lines (13 renal cancers, 5 melanomas, gliomas neuroblastomas, 15 epithelial cancers 5 B-cell lines K562 (an erythroid leukemia), 2 T-cell lines (MOLT-4 and T-45), and monkey kidney cells (VERO)). In addition, the antibodies were tested against short-term cultures of normal kidney epithelium, fibroblasts, and fetal tissues (brain, fibroblasts, and kidney). Human, sheep, rat and bovine erythrocytes were also examined. In most cases, serological analysis consisted of both direct and absorption tests.

These serological studies in conjunction with immunochemical analysis defined nine distinct antigenic systems. Three systems (gp160, S$_{25}$, and gp120) were restricted to normal and malignant renal cells, three systems (gp120nr, gp115, and V$_1$) were more widely distributed, and three systems werde identified as HLA-A, -B, C heavy chain and A and B blood group antigens.

In frozen section (Table II) S4, S22 and S6 show specificity for kidney tumors.

Description - Antigenic Systems

gp160 Antigenic System. Five Abs in this series (S$_4$, S$_7$, S$_{11}$, S$_{24}$, and M$_1$) identify a 160,000-dalton glycoprotein that showed a high degree of specificity for human kidney cells. gp160 is a rather basic component with pI 7.5. By M-MHA tests, gp16o could be demonstrated on all cultures of normal kidney epithelium, 2 of 3 cultures of fetal kidney, and 7 of 13 established lines of renal cancer (Table 2). These results were confirmed in absorption tests. No other cell type, normal or malignant, was found to express the gp160 antigen, including VERO, a cell line derived from monkey kidney.

S$_{25}$ Antigenic System. The antigen detected by AD S$_{25}$ also is restricted to human cells of renal origin (Table 2). The S$_{25}$ determinant is heat labile, suggesting that it resides on a protein or glucoprotein, but Ab S$_{25}$ did not precipitate any detectable component from ($^{35}$S) methionine-labeled or ($^3$H) glucosamine-labeled SK-RC-7 cells. Comparison of the S$_{25}$ and the gp160 phenotypes of different renal cancer lines and cultures of normal kidney clearly distinguished these two systems. For example, SK-RC-6 and A-498 are gp160$^+$ / S$_{25}^-$ and SK-RC-8 is gp160$^-$ / S$_{25}^+$, whereas five of these cultures lacked S$_{25}$ expression.

gp120r and gp120nr Antigenic Systems

Five Abs (S$_{23}$, S$_{26}$, S$_{27}$, S$_6$, and S$_1$) immunoprecipitated a 120,000-dalton glycoprotein from ($^{35}$S) methionine- or ($^3$H) glucosamine-labeled lysates of SK-RC-7 cells. Analysis under reducing and nonreducing conditions gave the same results. The pIs of gp120 identified by prototype Ab S$_6$ and Ab S$_{23}$ were identical (4.9-5.2). A further indication of the relatedness of the gp120 components identified by these two groups of Abs came from sequential immunoprecipitation tests. Pretreatment of ($^3$H) glucosamine-labeled lysates of SK-RC-7 with Ab S$_6$ removed all antigen reactive with Ab S$_{23}$. In contrast, M-MHA tests and absorption analysis (Table 2) showed that these gp120 antibodies identified two serologically distinct gp120 epitopes that distinguish two classes of gp120 molecules: gp120r (restricted) and gp120nr (nonrestricted).

gp120r, identified by Ab S$_{23}$, had a highly restricted distribution, expression being limited to normal kidney epithelium and certain renal cancers. The other gp120 epitope, gp120nr, identified by Ab S$_6$, was found on a wide range of cultured cells including fetal and adult fibroblasts and cell lines derived from ovarian, bladder and colon cancers. gp120r and gp120nr determinants differ in their expression on renal cancer cell lines: all cell lines carry the gp120nr epitope,whereas SK-RC-2 -21, -29, and Caki-1 lack gp120r determinants. The specificity of Ab S$_{23}$ for cells of renal origin resembles the reactivity of Ab S$_{25}$ and, most particularly, antibodies identifying the gp160 system. However, in addition to the molecular weight differences in the gp160 and gp120 antigens, these three kidney-specific antigenic systems can be distinguished on the basis of absorption analysis with selected normal or malignant kidney cells - e. g., SK-RC-6 and A-498 are are gp160$^+$/S$_{25}^-$/gp120r$^+$; fetal kidney is gp160$^+$ or $^-$/S$_{25}^+$/gp120r$^-$.

6

gp115 Antigenic System. Ab $S_{22}$ immunoprecipitated a 115,000-dalton glycoprotein from ($^3$H) glucosamine- or ($^{35}$S) methionine-labeled lysates of SK-RC-7 cells under both reduced and nonreduced conditions (Fig. 1). In direct M-MHA tests, high reactivity (titers, 1-10,000 x $10^{-3}$) was restricted to certain renal cancer cells and normal kidney epithelium (Table 2). Absorption analysis, however, revealed that the gp115 antigen was expressed by various cell types.

$V_1$ Antigenic System. Ab $V_1$ did not immunoprecipitate any labeled component from ($^3$H) glucosamine- or ($^{35}$S) methionine-labeled lysates of SK-RC-7 cells. Absorption tests indicated that the antigen is heat stable (5 mins. at 100ºC), suggesting that it is a glycolipid. Two features of the $V_1$ (Table 2) system are of particular interest: (a) it identifies a subset of bladder and breast cancers which do not express $V_1$, and (b) $V_1$ is not found on astrocytomas, whereas melanomas are strong $V_1$ expressors. This clear distinction between astrocytomas and melanoma, whose embryonic derivations are closely related, has not been seen with other Abs.

HLA Heavy Chain. Ab $S_{21}$ immunoprecipitated a 45,000- and a 12,000-dalton component from ($^{35}$S) methionine-labeled SK-RC-7 lysates. The determinant detected by Ab $S_{21}$ in direct and absorption tests was present on virtually every human cell type with the exception of human erythrocytes (Table 2). Of all the human cultured cells tested, the only cell lines not reactive with Ab $S_{21}$ in direct MHA tests were ME-180 and SK-MEL-19; the SK-MEL-19 melanoma cell line is known from previous work to express little or no HLA-A, -B, -C antigens. The molecular weights of the components precipitated by Ab $S_{21}$ and the results of the serological survey of human cells indicated that Ab $S_{21}$ detected HLA but did not distinguish between a determinant on the heavy chain or on the $_2$m chain. The fact that isolated human $_2$m did not inhibit the reactivity of AB $S_{21}$ suggests specificity for HLA heavy chain.

A and B Blood Group Antigens. Two of the three renal cancer lines used for immunization (Table 1) express blood group A and B antigens on their cell surfaces; SK-RC-7 is B$^+$ and SK-RC-28 is A$^+$. SK-RC-6 is derived from a type O individual and is negative for A and B reactivities. To detect Abs reacting with blood group antigens, hybridoma supernatants were screened for hemagglutinating antibody by using A, B, AB, or O erythrocytes. B (but not A) agglutinating activity was found in 4 of 462 supernatants from the anti-SK-RC-7 (fusion) and A (but not B) agglutinating activity was found in 3 of 225 supernatants from the anti-SK-RC-28 fusion. No agglutination of type O erythrocytes was found in supernatants from anti-SK-RC-7, -28 or -6 fusions. Two monoclonal antibodies with hemagglutinin activity were derived from these fusions. The hemagglutination titer of Ab $M_2$ (nu/nu serum) for A and AB erythrocytes was $10^{-4}$; B erythrocytes were not agglutinated by Ab $M_2$. The hemagglutination titer of Ab $S_8$ (nu/nu serum) for B and AB erythrocytes was 4 x $10^{-5}$; A type erythrocytes were not agglutinated by Ab $S_8$. Table 3 summarizes inhibition tests with Ab $S_8$ and Ab $M_2$ using glycoprotein and mucin extracts having A, B, H and Lewis$^a$ blood group reactivity. The results confirmed the A specificity of Ab $M_2$ and the B specificity of Ab $S_8$.

It is not always possible to predict successful hybridoma preparation of one cell line although success may have been achieved with another cell line. Any new cell line is impossible to predict because of the vagaries of the art of monoclonal antibodies. Preparation of hybrid cell lines can be successful or not depending on such experimental factors as nature of the inoculant, cell growth conditions, hybridization conditions and the like.

The present invention describes a new monoclonal antibody, $F_{23}$, a gamma sub 2A ($\gamma$ 2A) immunoglobulin (Ig). In the previous work mAb $S_4$ was in that same class of Ig while $S_{25}$, $S_{22}$, $S_{23}$, $S_6$, $S_{27}$, $V_1$ and $S_{21}$ belonged to immunoglobulin class gamma sub one ($\gamma_1$) and $M_2$ and $S_8$ to Ig class mu(u). $F_{23}$ recognizes a new antigenic system on human renal cells glycoprotein (gp) 140.

$F_{23}$ is derived from a hybridoma cell line wherein normal human epithelial cells are the immunogen. Yet antibodies to renal tumors are made by this invention. This is an unexpected result.

As can be seen from Table I, $F_{23}$ recognizes human renal cancer cell lines. Of 25 cell lines tested, $F_{23}$ is positioned for 19 of those. 33 human renal cell lines wer studied. $F_{23}$ also recognizes some poorly differentiated renal carcinomas and some renal carcinomas with papillary differentiation with its best reaction with well differentiated renal carcinomas i. e. $F_{23}$ subsets renal carcinomas and can be used to assay for the malignant potential of renal tumors. Frozen renal carcinoma section for over 20 different human specimens were tested as well. Comparison of frozen sections and tissue culture lines established from the same specimens reveals that for most antigens, expression is consistent in vivo and in vitro. See tables I & II.

Some antigens may be either induced or suppressed in tissue culture. This characteristic must be determined for each antigen prior to extrapolating results between in vivo and in vitro systems.

Therefore $F_{23}$ mAb must be added to the mAb panel for human renal cancer which to date includes then $F_{23}$, $M_1$, $M_2$, $S_1$, $S_4$, $S_6$, $S_7$, $S_8$, $S_{11}$, $S_{21}$, $S_{22}$, $S_{23}$, $S_{24}$, $S_{25}$, $S_{26}$, $S_{27}$, $V_1$ and $V_2$. This entire array of mAbs is used to diagnose renal cancer. A specimen tissue, body waste or fluid or exudate is contacted separately with each of the above mAbs in a screening test for a positive reaction.

$F_{23}$ also gives a positive reaction with two cell lines of normal kidney epithelium. However, $F_{23}$ did not react with normal human A, B or D erythrocytes by the absorption test. In frozen reactions, $F_{23}$ reacted with normal kidney and proximal tubule in fetal as well as adult specimens. $F_{23}$ showed a positive reaction in brain and neurons. Table II.

Normal proximal tubule cells express gp antigens at consistent levels (gp160, gp120r and gp115) whereas only 5/31 tumors expressed all three antigens. 26 tumors did not express these antigens and 8 of the tumor cell lines tested were negative for gp140. In further differentiating normal versus malignant cells it is noted that when one of the gp antigens (160, 120r or 115) was present in a tumor levels of expression of the antigen ranged as high as 10,000 x normal. Results show antigen expression correlated with the malignant potential of these tumors.

The above examples are for illustrative purposes only and are not meant to limit the scope of the invention. It is obvious that the invention also encompasses all monoclonal antibodies possessing the same characteristics as described herein. The examples do not limit the invention especially with respect to substantially functional equivalents of hybridomas, monoclonal antibodies and cell lines described and claimed herein.

Changes in cell antigens are associated with different stages of differentiation and different stages of cancer. Thus this invention technique defined cell antigens associated with differentiation and cancer of the kidney and its associated tubules.

In sum then the antibodies discussed subset the nephron and subset renal cancers. Methods used to obtain $F_{23}$ are as described above in the above section on techniques.

Micro-Organisms

The invention involves the use of a micro-organism (monoclonal antibody), which is deposited at the American Type Culture Collection (ATCC)
12301 Parklawn Drive
Rockville, Maryland 20852
U S A
and has been given ATCC Accession number HB 8231.

Legend  to Table I

Serological Reaction of monoclonal antibodies of Ueda et al supra (*) and F23 mAb with tumor cell lines and normal cell lines.

        0 = no reaction either by absorption or rosette formation

        θ = reaction by absorption only

        ◍ = reaction by rosette formation titer < 10,000

        ◕ = reaction by rosette formation titer > 10,000

        △ = no reaction – only absorption test done

        ▲ = reaction with absorption test only

Legend  to Table II

Immunopathological reaction of monoclonal antibodies of Ueda et al supra (*) and F23 mAb with frozen normal human tissue sections by immunofluorescence.

For parts A & B:

0 = no reaction

0 = heterogeneous positive reaction within the tissue

● = homogeneous positive reaction within the tissue

For parts C & D - frozen sections of human tumors

— or O = no reaction

± = heterogeneous reaction

+ = positive reaction

10

Note: C68 = colon monoclonal antibody CLH68

C26 = colon monoclonal antibody HT29/26

AJ8 = astrocytoma monoclonal antibody (Cairncross, et al. Proc. Nat'l. Acad. Sci. U.S.A. 1982)

NL-1 = monoclonal antibody recognizing calla antigen in acute lymphocytic leukemia (Tanimoto)

NL-22 = mAb (Tanimoto)

Q-14 = melanoma antibody

P170 = AJ2 astrocytoma (Cairncross, _Supra_)

P130 = AJ2 astrocytoma (Cairncross, Supra)

$S_6/S_{27}$ refers to test done using either $S_6$ or $S_{27}$ mAb which gives same reaction.

TABLE I

SEROLOGICAL REACTION OF mAbs PRODUCED FROM
HUMAN RENAL TUMORS AND NORMAL EPITHELIUM AS IMMUNOGEN
WITH VARIOUS CANCER CELL LINES AND NORMAL CELL LINES

| | S4 | S25 | S22 | S23 | S6 | S27 | V1 | S21 | F23 | | | | M2 | S8 |
|---|---|---|---|---|---|---|---|---|---|---|---|---|---|---|
| Ig class of antibody: | Y2a | Y1 | Y1 | Y1 | Y1 | Y1 | Y1 | Y1 | Y2a | | | | μ | μ |
| Antigen detected: | gp 160 | | gp 115 | gp 120r | gp 120nr | gp 120nr | | gp45 82 | gp 140 | | | | | |
| CELLS TESTED Renal cell lines | | | | | | | | | | | | | | |
| SK-RC-1 | ● | ⊙ | ⊙ | ① | ● | ● | ● | ● | | | | | | |
| SK-RC-2 | ○ | ○ | ⊙ | ○ | ● | ● | ● | ● | | | | | | |
| SK-RC-4 | ● | ● | ● | ● | ● | ● | ● | ● | ○ | | | | ○ | ① |
| SK-RC-6 | ● | ○ | ① | ● | ● | ● | ● | ● | | | | | | |
| SK-RC-7 | ● | ● | ● | ① | ● | ● | ● | ● | ⊙ | | | | ○ | ① |
| SK-RC-8 | ○ | ① | ● | ① | ● | ● | ● | ● | | | | | | |
| SK-RC-9 | ○ | ⊙ | ● | ⊙ | ● | ● | ● | ● | | | | | | |
| SK-RC-11 | ① | ⊙ | ⊙ | ① | ● | ● | ● | ● | | | | | | |
| SK-RC-12 | ● | | ○ | ① | | ● | | | ○ | | | | | |
| SK-RC-13 | ● | | ○ | ① | | ● | | | ○ | | | | | |
| SK-RC-15 | ● | | ○ | ● | | ● | | | ○ | | | | | |
| SK-RC-16 | ① | | ○ | ⊙ | | ● | | | ○ | | | | | |
| SK-RC-17 | ○ | | ○ | ○ | | ● | | | ① | | | | | |
| SK-RC-18 | ① | | ○ | ○ | | ● | | | ① | | | | | |
| SK-RC-20 | ⊙ | | ○ | ⊙ | | ● | | | ⊙ | | | | | |
| SK-RC-21 | ○ | ○ | ⊙ | ○ | ● | ● | ① | ● | ⊙ | | | | | |
| SK-RC-24 | ● | | ○ | ① | | ● | | | ● | | | | | |
| SK-RC-26a | ① | | ⊙ | ⊙ | | ● | | | ○ | | | | | |
| SK-RC-26b | ● | | ○ | ⊙ | | ● | | | ○ | | | | | |
| SK-RC-28 | ● | ⊙ | ⊙ | ● | ● | ● | ● | ● | | | | | ① | ○ |
| SK-RC-29 | ○ | ○ | ⊙ | ○ | ● | ● | ● | ● | | | | | | |
| SK-RC-33 | ① | | ① | ① | | ● | | | ● | | | | | |
| SK-RC-34 | ● | | ● | ● | | ● | | | ① | | | | | |
| SK-RC-35 | ● | | ● | ● | | ● | | | ● | | | | | |

12

TABLE I   (Cont'd.)

ANTIBODY

| CELLS TESTED<br>Renal cell lines | *S4 | *S25 | *S22 | *S23 | *S6 | *S27 | *V1 | *S21 | F23 | | | | | *M2 | *S8 | |
|---|---|---|---|---|---|---|---|---|---|---|---|---|---|---|---|---|
| SK-RC-37 | ⊙ | | ○ | ⊙ | -- | ◑ | | | ① | | | | | | | |
| SK-RC-38 | ◑ | | ○ | ◑ | | ◑ | | | ① | | | | | | | |
| SK-RC-39 | ◑ | | ⊙ | ◑ | | ◑ | | | ◑ | | | | | | | |
| SK-RC-40 | ◑ | | ① | ◑ | | ◑ | | | ◑ | | | | | | | |
| SK-RC-41 | ① | | ○ | ◑ | | ◑ | | | ① | | | | | | | |
| SK-RC-42 | ○ | | ○ | ○ | | ◑ | | | ① | | | | | | | |
| SK-RC-44 | ◑ | | ⊙ | ① | | ◑ | | | ⊙ | | | | | | | |
| SK-RC-45 | ◑ | | ○ | ◑ | | ◑ | | | ◑ | | | | | | | |
| SK-RC-46 | ◑ | | ○ | ○ | | ◑ | | | ○ | | | | | | | |
| A-498 | ◑ | ○ | ⊙ | ⊙ | ◑ | ◑ | ◑ | ◑ | | | | | | | | |
| CaKi-1 | ○ | ○ | ⊙ | ○ | ◑ | ◑ | ◑ | ◑ | | | | | | | | |
| Normal kidney epithelium | | | | | | | | | | | | | | | | |
| NK-LI | ◑ | | ① | ① | | ◑ | | | ① | | | | | | | |
| NK-LD | ① | | ○ | ⊙ | | ◑ | | | ⊙ | | | | | | | |
| NK-ID | | ○ | ① | ○ | ① | ① | ① | ◑ | | | | | | | | |
| NK-EQ | ◑ | ○ | ① | ○ | ① | ① | ① | ◑ | | | | | | | | |
| NK-HY | ◑ | ○ | ① | ○ | ① | ① | ① | ◑ | | | | | | | | |
| NK-GH | ① | ① | ① | ○ | ① | ① | ① | ◑ | | | | | | | | |
| NK-FR | ① | ① | ① | ○ | ① | ① | ① | ◑ | | | | | | | | |
| NK-EI | ① | ⊙ | ① | ○ | ① | ① | ① | ◑ | | | | | | | | |
| NK-IJ | ① | ⊙ | ① | ○ | ① | ① | ① | ◑ | | | | | | | | |
| NK-EG | ① | ○ | ① | ○ | ① | ① | ① | ◑ | | | | | | | | |
| NK-GR | ① | ○ | ① | ○ | ① | ① | ① | ◑ | | | | | | | | |
| NK-IB | ① | ○ | ① | ○ | ① | ① | ① | ◑ | | | | | | ○ | ① | |
| Normal fetal kidney cells | | | | | | | | | | | | | | | | |
| FK-C4 | ① | ① | ① | ○ | ① | ① | ① | ◑ | | | | | | | | |
| FK-C6 | ○ | ① | ① | ○ | ① | ① | ① | ◑ | | | | | | | | |
| FK-C8 | ① | ① | ① | ○ | ① | ① | ① | ◑ | | | | | | | | |

13

TABLE I    (Cont'd)

ANTIBODY

| CELL TESTED Epithelial cancer cell | *S4 lines | *S25 | *S22 | *S23 | *S6 | *S27 | *V1 | *S21 | F23 | | | | *M2 | *S8 |
|---|---|---|---|---|---|---|---|---|---|---|---|---|---|---|
| Bladder cell lines | | | | | | | | | | | | | | |
| *RT-4 | O | O | ⊙ | O | O | O | ⊙ | ⊕ | | | | | | |
| *5637 | O | O | ⊙ | O | O | O | O | ⊕ | | | | | | |
| *T-24 | O | O | ⊙ | O | ① | ① | O | ⊕ | | | | | | |
| *253J | O | O | ⊙ | O | ① | ① | ⊕ | ⊕ | | | | | ① | O |
| Breast cell lines | | | | | | | | | | | | | | |
| *A1Ab | O | O | O | O | O | O | ① | ⊕ | | | | | | |
| *BT-20 | O | O | O | O | O | O | O | ⊕ | | | | | | |
| * | O | O | O | O | O | O | ⊕ | ⊕ | | | | | | |
| *SK-BR-3 | O | O | O | O | O | O | ⊕ | ⊕ | | | | | O | ① |
| Cervix cell line | | | | | | | | | | | | | | |
| *ME-180 | O | O | O | O | O | O | ⊙ | ⊙ | | | | | ① | O |
| Colon cell lines | | | | | | | | | | | | | | |
| *HT-29 | O | O | O | O | ① | ① | ⊙ | ⊕ | | | | | ① | O |
| *SW-1222 | O | O | O | O | O | O | ⊕ | ① | | | | | ① | O |
| Lung cell lines | | | | | | | | | | | | | | |
| *SK-LC-LL | O | O | ⊙ | O | O | O | ① | ① | | | | | | |
| *SK-LC-6 | O | O | O | O | ⊕ | ⊕ | ⊕ | ⊕ | | | | | | |
| Ovary cell line | | | | | | | | | | | | | | |
| *SK-OV-3 | O | O | ① | O | O | O | ⊙ | ⊕ | | | | | | |
| Normal cell lines | | | | | | | | | | | | | | |
| *Adult skin fibroblas | O | O | O | O | ① | ① | O | ① | | | | | | |
| *Fetal lung fibroblas | O | O | ① | O | ① | ① | ① | ⊕ | ⊕ | | | | | |

14

TABLE I   (Cont'd)

ANTIBODY

| CELLS TESTED | *S4 | *S25 | *S22 | *S23 | *S6 | *S27 | *V1 | *S21 | V23 | | | | *M2 | *S8 |
|---|---|---|---|---|---|---|---|---|---|---|---|---|---|---|
| Neuroectoderm cancer cell lines | | | | | | | | | | | | | | |
| Astrocytoma | | | | | | | | | | | | | | |
| * SK-MG-1 | ○ | ○ | ⊙ | ○ | ○ | ○ | ○ | ● | | | | | | |
| * SK-MG-4 | ○ | ○ | ⊙ | ○ | ○ | ○ | ○ | ● | | | | | | |
| * SK-MG-7 | ○ | ○ | ⊙ | ○ | ○ | ○ | ○ | ● | | | | | | |
| Melanoma | | | | | | | | | | | | | | |
| * SK-MEL-13 | ○ | ○ | ○ | ○ | ○ | ○ | ● | ● | | | | | | |
| * SK-MEL-19 | ○ | ○ | ○ | ○ | ○ | ○ | ● | ⊙ | | | | | | |
| * SK-MEL-28 | ○ | ○ | ○ | ○ | ○ | ○ | ● | ● | | | | | | |
| * SK-MEL-29 | ○ | ○ | ○ | ○ | ○ | ○ | ● | ● | | | | | | |
| * SK-MEL-37 | ○ | ○ | ○ | ○ | ○ | ○ | ● | ● | | | | | | |
| * SK-MEL-41 | ○ | ○ | ○ | ○ | ○ | ○ | ● | ● | | | | | | |
| Neuroblastoma | | | | | | | | | | | | | | |
| * SK-NMC | ○ | ○ | ○ | ○ | ○ | ○ | ◑ | ● | | | | | | |
| * SK-NSH | ○ | ○ | ○ | ○ | ○ | ○ | ◑ | ● | | | | | | |
| Normal primary cultures | | | | | | | | | | | | | | |
| * Fetal brain | ○ | ○ | △ | ○ | △ | △ | △ | ● | | | | | | |
| ∮ Fetal melanocytes | ○ | ○ | ○ | ● | ● | | ● | ● | | | | | | |
| ∮ Adult melanocytes | ○ | ○ | ○ | ● | ● | | ● | ● | | | | | | |

TABLE I (Cont'd)

ANTIBODY

| CELLS TESTED | S4* | S25* | S22* | S23* | S6* | S27* | V1* | S21* | F23 | | | | M2* | S8* | |
|---|---|---|---|---|---|---|---|---|---|---|---|---|---|---|---|
| **Hematopoetic cancer cell lines** | | | | | | | | | | | | | | | |
| *Lymphoblastoid cells* | | | | | | | | | | | | | | | |
| EBV B cells - AX | △ | △ | △ | △ | △ | △ | ▲ | △ | | | | | | | |
| EBV B cells - BE | △ | △ | △ | △ | △ | △ | ▲ | △ | | | | | | | |
| EBV B cells - EU | △ | △ | △ | △ | △ | △ | ▲ | △ | | | | | | | |
| Burkitt's Lym - Daudi | △ | △ | △ | △ | △ | △ | ▲ | △ | | | | | | | |
| Burkitt's Lym - Raji | △ | △ | △ | △ | △ | △ | ▲ | ▲ | | | | | | | |
| T cells - MOLT-4 | △ | △ | △ | △ | △ | △ | △ | △ | | | | | | | |
| cells - T-45 | △ | △ | △ | △ | △ | △ | △ | △ | | | | | | | |
| **Normal cells** | | | | | | | | | | | | | | | |
| Erythrocytes - A | △ | △ | △ | △ | △ | △ | △ | △ | △ | | | | ● | ○ | |
| Erythrocytes - B | △ | △ | △ | △ | △ | △ | △ | △ | △ | | | | ○ | ● | |
| Erythrocytes - 0 | △ | △ | △ | △ | △ | △ | △ | △ | △ | | | | ○ | ○ | |
| | | | | | | | | | | | | | | | |
| **Xenogeneic cells** | | | | | | | | | | | | | | | |
| Monkey kidney - VERO | ○ | ○ | ○ | ○ | ● | ○ | ○ | ○ | | | | | | | |
| Sheep erythrocytes | △ | △ | △ | △ | △ | △ | △ | △ | | | | | ○ | ○ | |

References

\* Ueda, R., Ogata, S.-I., Morrissey, D.M., Finstad, C.L., Szkudlarek, J., Whitmore, W.F., Oettgen, H.F., Lloyd, K.O. and Old, L.J. Cell surface antigens of human renal cancer defined by mouse monoclonal antibodies: Identification of tissue specific kidney glycoproteins. Proc. Nat. Acad. Sci. 78:5122-5126 (1981).

♪ Houghton, A.N., Eisinger, M. Albino, A.P., Cairncross, J.G. and Old, L.J. Surface antigens of melanocytes and melanomas. Markers of melanocyte differentiation and melanoma subsets.

TABLE II

TISSUE DISTRIBUTION OF THE MONOCLONAL ANTIBODIES OF THE KIDNEY

A. FETAL TISSUES (Normal)

| | S4 | SG/27 | S23 | F23 | S22 | Q14 | AJ8 | NL-1 | NL-22 | P170-140 | C26 | C68 |
|---|---|---|---|---|---|---|---|---|---|---|---|---|
| LUNG | O | O | O | O | O | O | O | O | O | O | ● | O |
| Bronchial Epithelium | O | O | O | O | O | O | O | O | O | O | ● | O |
| Cartilage | O | O | O | O | O | O | O | O | O | O | O | C |
| Pneumocytes | O | O | O | O | O | O | O | O | O | O | O | O |
| Connect.Tis | O | O | O | O | O | O | O | O | O | O | O | C |
| HEART | O | O | O | O | O | O | O | O | O | O | O | O |
| THYMUS | O | O | O | O | O | O | O | O | O | O | O | ● |
| Hassal's C. | O | O | O | O | O | O | O | O | O | O | O | O |
| Thymocytes | O | O | O | O | O | O | O | O | O | O | O | O |
| SPLEEN | O | O | O | O | O | O | O | O | O | O | O | O |
| White Pulp | O | O | O | O | O | O | O | O | O | O | O | O |
| Red Pulp | O | O | O | O | O | O | O | O | O | O | O | O |
| LIVER. | O | O | O | O | O | O | O | O | O | O | ● | ● |
| Hepatocytes | O | O | O | O | O | O | O | O | O | O | O | C |
| Biliary Epi Cells | O | O | O | O | O | O | O | O | O | O | ● | ● |
| GALLBLAD. | O | O | O | O | O | O | O | O | O | O | ● | ● |
| ESOPHAGUS | O | O | O | O | O | O | O | O | O | O | O | O |
| STOMACH | O | O | O | O | O | O | O | O | O | O | O | ● |
| SMALL INT. | O | O | O | O | O | O | O | O | O | O | O | ● |
| COLON | O | O | O | O | O | O | O | O | O | O | ● | ● |
| PANCREAS | O | O | O | O | O | O | O | O | O | O | ● | C |
| Exocrine | O | O | O | O | O | O | O | O | O | O | ● | C |
| Endocrine | O | O | O | O | O | O | O | O | O | O | O | C |
| KIDNEY | ● | ● | ● | ● | O | ● | ● | ● | O | ● | ● | C |
| Glomerulus | ● | O | O | O | O | ● | ● | ● | O | ● | O | C |
| Prox. Tub. | ● | ● | ● | ● | O | O | ● | ● | O | O | O | C |
| Distal Tub. | O | O | O | O | O | O | O | O | O | O | ● | C |
| Collec. Tub | O | O | O | O | O | O | O | O | O | O | O | C |
| URETER | O | ● | O | O | O | O | O | O | O | O | ● | ● |
| UR. BLAD. | O | ● | O | O | O | O | O | O | O | O | ● | ● |
| ADRENAL | O | O | O | O | O | O | O | O | O | O | O | C |
| Cortex | O | O | O | O | O | O | O | O | O | O | O | C |
| Medulla | O | O | O | O | O | O | O | O | O | O | O | C |
| TESTES | O | O | O | O | O | O | O | O | O | O | O | O |
| Germ. Cells | O | O | O | O | O | O | O | O | O | O | O | C |
| Endoc. Cel. | O | O | O | O | O | O | O | O | O | O | O | O |
| Connect. T. | O | O | O | O | O | O | O | O | O | O | O | O |

A. FETAL TISSUES (Normal)    (Cont'd.)

| | S4 | S6/27 | S23 | F23 | S22 | Q14 | AJ8 | NL-1 | NL-22 | P170/148 | C26 | CG2 |
|---|---|---|---|---|---|---|---|---|---|---|---|---|
| OVARY | O | O | O | O | O | O | O | O | O | O | O | O |
| Germ. Cells | O | O | O | O | O | O | O | O | O | O | O | O |
| Connect. T. | O | O | O | O | O | O | O | O | O | O | O | O |
| FALLOP. T. | O | O | O | O | O | O | O | O | O | O | ● | O |
| UTERUS | O | O | O | O | O | O | O | O | O | O | ● | O |
| Endometrium | O | O | O | O | O | O | O | O | O | O | ● | O |
| Myometrium | ● | O | O | O | O | O | O | O | O | O | O | O |
| CERVIX | O | O | O | O | O | O | O | O | O | O | ● | ● |
| Endocervix | O | O | O | O | O | O | O | O | O | O | ● | O |
| Exocervix | O | O | O | O | O | O | O | O | O | O | O | ● |
| SKIN | O | O | O | O | O | O | O | O | O | O | ● | ● |
| Epidermis | O | O | O | O | O | O | O | O | O | O | O | ● |
| Melanocytes | O | O | O | O | O | O | O | O | O | O | O | O |
| Sweat Gland | O | O | O | O | O | O | O | O | O | O | ● | O |
| Sebac. Gld. | O | O | O | O | O | O | O | O | O | O | O | O |
| Hair Fol. | O | O | O | O | O | O | O | O | O | O | O | O |
| Dermis C.T. | O | O | O | O | O | O | O | O | O | O | O | O |
| BRAIN | O | O | O | O | O | O | ● | O | O | O | O | O |
| Neurons | O | O | O | O | O | O | ● | O | O | O | O | O |
| Glial Cells | O | O | O | O | O | O | O | O | O | O | O | O |
| Dendrites | O | O | O | ● | O | O | ● | O | O | O | O | O |
| LYMPH NODE | O | O | O | O | O | O | O | O | O | O | O | O |
| BLOOD VES. | ● | O | O | O | O | ● | O | O | O | O | O | O |
| Endoth. Cel. | O | O | O | O | O | ● | ● | O | O | O | O | O |
| Smooth Ms. | ● | O | O | O | O | O | O | O | O | O | O | O |
| SOFT TIS. | O | O | O | O | O | O | ● | O | O | ● | O | O |
| SECRETION | O | O | O | O | O | O | O | O | O | O | ● | O |

18

B. ADULT TISSUES (Normal)

| | S4 | S6/27 | S23 | F23 | S22 | Q14 | AJ8 | NL-1 | NL-22 | P170 140 | C26 | C68 |
|---|---|---|---|---|---|---|---|---|---|---|---|---|
| LUNG | O | O | O | O | O | O | O | O | O | O | ● | O |
| Bronchial Epithelium | O | O | O | O | O | O | O | O | O | O | ● | O |
| Cartilage | O | O | O | O | O | O | O | O | O | O | O | O |
| Glandular Epithelium | O | O | O | O | O | O | O | O | O | O | ● | O |
| Pneumocytes | O | O | O | O | O | O | O | O | O | O | O | O |
| Connect. Tis | O | O | O | O | O | O | O | O | O | O | O | O |
| HEART (ms) | O | O | O | O | O | O | O | O | O | O | O | O |
| SPLEEN | O | O | O | Q | O | O | O | O | O | O | O | O |
| White pulp | O | O | O | O | O | O | O | O | O | O | O | O |
| Red pulp | O | O | O | O | O | O | O | O | O | O | O | O |
| LIVER | O | O | O | O | O | O | O | O | O | O | ● | O |
| Hepatocyte | O | O | O | O | O | O | O | O | O | O | O | O |
| Bil. Epit. | O | O | O | O | O | O | O | O | O | O | ● | O |
| Sinusoids | O | O | O | O | O | O | O | O | O | O | O | O |
| GALLBLADDER | O | O | O | O | O | O | O | O | O | O | ● | O |
| ESOPHAGUS | O | O | O | O | O | O | O | O | O | O | O | O |
| STOMACH | O | O | O | O | O | O | O | O | O | O | O | ● |
| SM. INTEST. | O | O | O | O | O | O | O | O | O | O | O | ● |
| COLON | O | ● | ● | O | O | O | O | O | O | O | ● | ● |
| G.I. Smc | O | O | O | O | O | O | O | O | O | O | O | O |
| PANCREAS | O | O | O | O | O | O | C | O | O | O | ● | O |
| Exocrine | O | O | O | O | O | O | C | O | O | O | ● | O |
| Endocrine | O | O | O | O | O | O | O | O | O | O | O | O |
| KIDNEY | ● | ● | ● | ● | O | ● | ● | ● | ● | ● | ● | O |
| Glomerulus | ● | O | O | O | O | ● | ● | ● | O | ● | O | C |
| Prox. Tub. | ● | ● | ● | ● | O | O | ● | ● | ● | O | O | C |
| Henle's L. | O | ● | O | O | O | O | ● | O | O | O | ● | O |
| Distal Tub. | O | O | O | O | O | O | O | O | O | O | ● | C |
| Collec. Tub. | O | O | O | O | O | O | O | O | O | O | ● | C |
| URETER | O | ● | O | O | O | O | O | O | O | O | ● | ● |
| URI. BLAD. | O | ● | O | O | O | O | O | O | O | O | ● | ● |
| ADRENAL | O | O | O | O | O | O | O | O | O | O | O | O |
| Cortex | O | O | O | O | O | O | O | O | O | O | O | O |
| Medulla | O | O | O | O | O | O | O | O | O | O | O | O |
| THYROID | O | O | O | O | O | O | O | O | O | O | O | O |
| Epitelium | O | O | O | O | O | O | O | O | O | O | O | O |
| Colloid | O | O | O | O | O | O | O | O | O | O | O | O |

TISSUE DISTRIBUTION OF THE MONOCLONAL ANTIBODIES OF THE KIDNEY

B. ADULT TISSUES (CONT'D)

| | S4 | S6/27 | S23 | F23 | S22 | Q14 | AJ8 | NL-1 | NL-22 | P170 140 | C26 | C68 |
|---|---|---|---|---|---|---|---|---|---|---|---|---|
| BREAST | O | O | O | O | O | O | O | O | O | O | ● | ● |
| Duct Cells | O | O | O | O | O | O | O | O | O | O | ● | ● |
| Acinar Cel. | O | O | O | O | O | O | O | O | O | O | ● | O |
| Connec.Tis. | O | O | O | O | O | O | O | O | O | O | O | O |
| PROSTATE | O | ● | ● | O | O | O | O | O | O | O | ● | ● |
| Epithelium | O | ● | ● | O | O | O | O | O | O | O | ● | ● |
| Stroma | O | O | O | O | O | O | O | O | O | O | O | O |
| TESTE | O | O | O | O | O | O | O | O | O | O | O | O |
| Germ Cells | O | O | O | O | O | ρ | O | O | O | O | O | O |
| Endocrine Cells | O | O | O | O | O | O | O | O | O | O | O | O |
| Connec.Tis. | O | O | O | O | O | O | O | O | O | O | O | O |
| OVARY | O | O | O | O | O | O | O | O | O | O | O | O |
| Germ Cells. | O | O | O | O | O | O | O | O | O | O | O | O |
| Connec.Tis. | O | O | O | O | O | O | O | O | O | O | O | O |
| FALLOP.TUB. | O | O | O | O | O | O | O | O | O | O | O | O |
| UTERUS | O | O | O | O | O | O | O | O | O | O | ● | O |
| Endometrium | O | O | O | O | O | O | O | O | O | O | ● | O |
| Myometrium | ● | O | O | O | O | O | O | O | O | O | O | O |
| CERVIX | O | O | O | O | O | O | O | O | O | O | ● | ● |
| Endocervix | O | O | O | O | O | O | O | O | O | O | ● | O |
| Exocervix | O | O | O | O | O | O | O | O | O | O | ● | ● |
| PLACENTA | O | O | O | O | O | O | O | O | O | O | O | O |
| Cytotrophb. | O | O | O | O | O | O | O | O | O | O | O | O |
| Syncytotrb. | O | O | O | O | O | O | O | O | O | O | O | O |
| Sinusoids | O | O | O | O | O | O | O | O | O | O | O | O |
| SKIN | O | O | O | O | O | O | O | O | O | O | ● | O |
| Epidermis | O | O | O | O | O | O | O | O | O | O | ● | O |
| Melanocytes | O | O | O | O | O | O | O | O | O | O | O | O |
| Sweat Gld. | O | O | O | O | O | O | O | O | O | O | ● | O |
| SebaceousG. | O | O | O | O | O | O | O | O | O | O | ● | O |
| Dermis CT | O | O | O | O | O | O | O | O | O | O | O | O |
| BRAIN | O | O | O | ● | O | O | ● | O | O | ● | O | O |
| Neurons | O | O | O | ● | O | O | ● | O | O | ● | O | O |
| Glial Cell | O | O | O | O | O | O | O | O | O | O | O | O |
| Dendrites | O | O | O | ● | O | O | O | O | O | O | O | O |
| LYMPH NODE | O | O | O | O | O | O | O | O | O | O | O | O |
| Fol/Medul | O | O | O | O | O | O | O | O | O | O | O | O |

TISSUE DISTRIBUTION OF THE MONOCLONAL ANTIBODIES OF THE KIDNEY

2

B. ADULT TISSUES (CONT'D.)

| | S4 | S6/27 | S23 | F23 | S22 | Q14 | AJ8 | NL-1 | NL-22 | P170 140 | C26 | C68 |
|---|---|---|---|---|---|---|---|---|---|---|---|---|
| BLOOD VES. | ● | O | O | O | O | ● | O | O | O | O | O | C |
| Endothelium | O | O | O | O | O | ● | O | O | O | O | O | O |
| Smooth Ms. | ● | O | O | O | O | O | O | O | O | O | O | O |
| CAPILLARIES | O | O | O | O | O | ● | O | O | O | O | O | O |
| SKELETAL MS | O | O | O | O | O | O | O | O | O | O | O | C |
| SOFT TISSUE | O | O | O | ● | O | O | O | O | O | ● | O | O |
| SECRETIONS | O | O | O | O | O | O | O | O | O | ● | ● | ● |

21

TABLE II

TISSUE DISTRIBUTION OF THE MONOCLONAL ANTIBODIES OF THE KIDNEY

C. RENAL CARCINOMAS

| | S6 | S9 | F23 | S23 | S22 | | S25 | S27 | C26 | T43 | T16 | T87 |
|---|---|---|---|---|---|---|---|---|---|---|---|---|
| RC #3 | + | +- | + | − | − | | − | | − | + | − | − |
| RC #5 | + | ± | ± | ± | − | | − | | ± | ± | ± | ± |
| RC #6 | + | + | + | + | ⊕ | | − | | − | ± | − | − |
| RC #2 | + | + | + | + | − | | − | | ± | − | − | ± |
| RC #7 | + | + | ± | + | − | | − | | − | ± | − | − |
| RC #9 | + | ± | − | + | − | | − | | − | − | − | − |
| RC #10 | + | − | − | + | ∓ | | − | | + | − | +, | + |
| RC #11 | + | + | ± | − | ∓ | | − | | ± | − | − | − |
| RC #12 | + | + | + | − | − | | − | | − | − | − | − |
| RC #13 | + | + | + | − | − | | − | | − | − | − | − |
| RC #22 | + | − | ± | − | − | | − | | ± | + | − | + |
| RC #23 | + | + | + | + | − | | − | | − | + | − | − |
| RC #14 | + | ± | − | − | − | | − | | − | − | − | − |
| RC #16 | + | + | ± | ± | − | | | | − | − | − | − |
| RC #19 | + | + | ± | + | − | | | + | − | + | − | − |
| RC #18 | + | − | − | − | − | | | | N/ | − | ±, | +- |
| RC #21 | + | + | ± | + | ± | | | + | − | − | | |
| RC #15 | +- | − | − | − | − | | | − | ± | + | | |
| Billoti (NSUH) | + | − | − | ± | − | | | + | − | ± | − | − |
| Pipet (NYU) | + | ± | ± | + | ⊕ | | | + | − | − | − | − |
| RC #17 | + | ± | ± | ± | ⊖ | | | + | ± | | ±, | ± |
| RC #20 | + | ± | ± | + | ⊕ | | | + | − | | − | − |
| RC #24 | + | − | ± | ⊕ | ⊕ | | | ± | − | | − | |
| RC #25 | − | − | − | − | − | | | − | − | − | − | − |

TISSUE DISTRIBUTION OF THE MONOCLONAL ANTIBODIES OF THE KIDNEY

C. Summary of tumor immunopathology staining using FITC-rabbit anti-mouse IgG

| | | κ(M)/N9 | S4 | S22 | F23 | S23 | S27 | S25 | V1 | C26 | T16 | AJ8 | HL |
|---|---|---|---|---|---|---|---|---|---|---|---|---|---|
| Colon | 82-18240 | - | - | - | - | +/- | - | + | - | + | - | - | - |
| | 83-153 | - | - | - | - | +/+++ | - | + | - | + | - | - | - |
| | 82-16115 | - | - | - | - | + | - | + | - | + | - | - | - |
| | 82-21302 | - | - | - | - | - | - | + | - | + | - | - | - |
| | invad.adenoca | - | - | - | - | - | - | + | - | + | - | - | - |
| Lung | 83-692 | - | - | - | - | - | - | + | - | + | - | - | - |
| | 83-2016 | - | - | - | + | -/+ | - | + | - | + | +w | - | - |
| | 83-323 | - | - | - | - | - | - | +w | - | + | - | - | - |
| | 83-337 | - | - | - | - | - | - | + | - | + | +w | - | - |
| | Oat cell ca | - | - | - | - | - | - | +? | - | + | - | - | - |
| Breast | fibroadenoma | - | - | - | -/(+) | - | - | -? | - | + | -/w | - | - |
| | 82-15096 | - | - | - | + | - | - | + | - | + | + | - | - |
| | 82-7783 | - | - | - | - | - | - | + | - | + | +w | - | - |
| | 82-14627 | - | - | - | - | - | - | +? | - | + | +w | - | - |
| | mst.to.lym.n. | - | - | - | - | - | - | + | - | +w | - | - | - |
| Bladder | papilloma | - | - | - | - | - | - | + | - | + | + | | |
| | Arnold/in situ | - | - | - | - | - | - | -? | - | - | + | - | - |
| | Grant TCC | - | - | -? | - | - | - | -? | - | - | + | - | - |
| | Wilson TCC | - | - | - | - | - | - | +? | - | +w | + | - | - |
| | Caurin TCC | - | - | +/- | - | - | - | + | - | + | +w | - | - |
| Teratoca | 82-20793 | - | - | - | + | + | + | -? | - | -w | + | + | + |
| | 83-1881a | - | - | - | - | - | - | + | - | + | - | - | - |
| | 83-1881b | - | - | - | - | - | - | + | - | - | - | - | - |
| | 82-19590 | - | - | - | - | - | - | + | - | + | - | - | - |
| Nevus | 82-23735 | - | - | - | + | - | - | + | - | - | - | - | - |
| Melanosac. | 82-16834 | + | - | - | + | - | - | + | - | - | - | - | - |
| Astrocytoma | grade I | - | - | - | + | - | - | - | - | - | - | - | - |
| | grade III | - | - | - | - | - | - | - | - | - | - | - | - |
| Lymphoma | 82-16027 | - | - | - | - | - | - | - | - | - | - | - | - |
| | 83-2542 | - | - | - | - | - | - | +? | - | - | - | - | - |
| | smooth muscle | + | - | - | - | - | - | -? | - | - | - | - | - |
| | endothelial c | + | - | - | - | - | - | -? | - | - | - | - | - |
| | interstitial | - | + | - | - | - | - | -? | - | - | - | - | - |
| | fibroblast | - | r | - | + | - | - | + | - | - | - | - | - |
| | connect.tis. | - | - | - | + | - | - | + | - | - | - | - | - |

## Claims

1. Monoclonal antibody-producing hybridoma cell line obtainable by fusing a myeloma derived cell strain and splenocytes derived from BALB/c mice immunized with human normal renal epithelial cells, said monoclonal antibody being capable of recognizing human renal epithelial cancer cells.

2. Monoclonal antibody produced by the hybridoma cell line of claim 1.

3. Monoclonal antibody which recognizes the human renal cell antigen gp140, said antigen gp140 being recognizable by the monoclonal reference antibody F23 (ATCC HB 8231).

4. Monoclonal antibody designated F23 which recognizes human renal cells and which is produced by the hybridoma cell line deposited with the ATCC under Accession No. HB 8231.

5. Hybridoma cell line according to claim 1 which produces the monoclonal antibody designated F23 and deposited with the ATCC under Acession No. HB 8231.

6. Method of forming a monoclonal antibody-producing hybridoma cell line by fusing a myeloma derived cell strain with splenocytes derived from BALB/c mice immunized with normal human renal epithelial cells.

7. Hybridoma cell line formed by the method of claim 6.

8. Monoclonal antibodies produced by the hybridoma cell line of claim 7.

9. Method for differentiating between normal and malignant human renal cells which comprises contacting a human renal sample with a plurality of monoclonal antibodies each of which is different and each of which is produced by a hybridoma cell line formed by fusing a myeloma derived cell strain and splenocytes derived from BALB/c mice immunized with established culture lines from the group of human epithelial renal cancer or normal human renal epithelial cells under suitable conditions so as to form a complex between each antibody and an antigen, detecting the presence of any so formed complex, the absence of any complex indicating normal renal cells and the presence of any complex indicating the presence of malignant human renal cells.

10. Method of claim 9, wherein the plurality of monoclonal antibodies is selected from the group consisting of $F_{23}$, $M_2$, $S_8$ and $S_{21}$.

11. Method of claim 10, which further comprises contacting a human renal sample with a plurality of monoclonal antibodies selected from the group consisting of $M_1$, $S_4$, $S_6$, $S_7$, $S_{22}$, $S_{23}$, $S_{24}$, $S_{25}$, $S_{26}$, $S_{27}$ and $V_1$.

12. Method of claim 10 wherein the method is used as an assay for the malignant potential of human renal tumors.

13. Antibody/antigen complex comprising monoclonal antibodies of any one of claims 2 to 4 and 8 and human renal cells.

14. Antibody/antigen complex according to claim 13 wherein the monoclonal antibodies are bound to antigenic human renal cell components.

**Revendications**

1. Lignée cellulaire d'hybridome produisant un anticorps monoclonal pouvant être obtenu par la fusion d'une souche cellulaire dérivée d'un myélome et de splénocytes dérivés de souris BALB/c immunisées avec des cellules éphitéliales rénales normales humaines, l'anticorps monoclonal pouvant reconnaître les cellules du cancer épithélial rénal humain.

2. Anticorps monoclonal produit par la lignée cellulaire de l'hybridome selon la revendication 1.

3. Anticorps monoclonal qui reconnaît l'antigène des cellules rénales humaines gp140, cet antigène gp140 pouvant être reconnu par l'anticorps de référence monoclonal F23 (ATCC HB 8231).

4. Anticorps monoclonal désigné F23 qui reconnaît les cellules rénales humaines et qui est produit par la lignée cellulaire de l'hybridome déposée à l'ATCC sous le numéro d'accession HB 8231.

24

**5.** Lignée cellulaire d'hybridome selon la revendication 1 qui produit l'anticorps monoclonal désigné F23 et déposée à l'ATCC sous le numéro d'accession HB 8231.

**6.** Procédé pour former une lignée cellulaire d'hybridome produisant l'anticorps monoclonal en fusionnant une souche cellulaire dérivée du myélome avec des splénocytes dérivés de souris BALB/c immunisées avec des cellules épithéliales rénales humaines normales.

**7.** Lignée cellulaire d'hybridome formée par le procédé selon la revendication 6.

**8.** Anticorps monoclonaux produits par la lignée cellulaire d'hybridome de la revendication 7.

**9.** Procédé pour la différenciation entre des cellules rénales humaines normales et malignes qui comprend les étapes consistant à mettre en contact un échantillon rénal humain avec plusieurs des anticorps monoclonaux dont chacun est différent et dont chacun est produit par une lignée cellulaire d'hybridome formée par la fusion d'une souche cellulaire dérivée d'un myélome et de splénocytes dérivés de souris BALB/c immunisées avec des lignées de culture établies à partir du groupe de cellules du cancer rénal épithélial humain ou de cellules épithéliales rénales humaines normales dans des conditions appropriées de façon à former un complexe entre chaque anticorps et un antigène, à détecter la présence de tout complexe ainsi formé, l'absence de tout complexe indiquant des cellules rénales normales et la présence de tout complexe indiquant la présence de cellules rénales humaines malignes.

**10.** Procédé selon la revendication 9, dans lequel on choisit plusieurs des anticorps monoclonaux dans le groupe constitué par $F_{23}$, $M_2$, $S_8$ et $S_{21}$.

**11.** Procédé selon la revendication 10, qui comprend de plus l'étape consistant à mettre en contact un échantillon rénal humain avec plusieurs des anticorps monoclonaux sélectionnés dans le groupe constitué par $M_1$, $S_4$, $S_5$, $S_7$, $S_{22}$, $S_{23}$, $S_{24}$, $S_{25}$, $S_{26}$, $S_{27}$, et $V_1$.

**12.** Procédé selon la revendication 10, dans lequel le procédé est utilisé comme analyse pour le potentiel malin, des tumeurs rénales humaines.

**13.** Complexe anticorps/antigène comprenant des anticorps monoclonaux selon l'une quelconque des revendications 2 à 4 et 8 et des cellules rénales humaines.

**14.** Complexe anticorps/antigène selon la revendication 13 dans lequel les anticorps monoclonaux sont liés aux composants cellulaires rénaux humains antigènes.

**Patentansprüche**

**1.** Monoclonalen Antikörper produzierende Hybridomzellinie, erhältlich durch Fusion eines von einem Myelom stammenden Zellstamms und von Splenozyten, die von mit normalen menschlichen Nierenepithelzellen immunisierten BALB/c-Mäusen stammen, wobei der monoclonale Antikörper fähig ist, menschliche Nierenepithelkrebszellen zu erkennen.

**2.** Monoclonaler Antikörper, produziert durch die Hybridomzellinie nach Anspruch 1.

**3.** Monoclonaler Antikörper, der das menschliche Nierenzellenantigen gp140 erkennt, wobei das Antigen gp140 durch den monoclonalen Bezugsantikörper F23 (ATCC HB 8231) erkennbar ist.

**4.** Monoclonaler Antikörper mit der Bezeichnung F23, der menschliche Nierenzellen erkennt und von der bei der ATCC unter der Hinterlegungsnummer HB 8231 hinterlegte Hybridomzellinie produziert wird.

**5.** Hybridomzellinie nach Anspruch 1, die den monoclonalen Antikörper mit der Bezeichnung F23, welcher bei der ATCC unter der Hinterlegungsnummer HB 8231 hinterlegt ist, produziert.

**6.** Verfahren zur Herstellung einer monoclonalen Antikörper produzierenden Hybridomzellinie durch Fusion eines von einem Myelom stammenden Zellstammes mit Splenozyten, die von mit normalen menschlichen Nierenepithelzellen immunisierten BALB/c-Mäusen stammen.

**7.** Hybridomzellinie, hergestellt durch das Verfahren nach Anspruch 6.

**8.** Monoclonale Antikörper, produziert durch die Hybridomzellinie nach Anspruch 7.

**9.** Verfahren zur Differenzierung zwischen normalen und malignen menschlichen Nierenzellen, umfassend das Inkontaktbringen einer Probe von einer menschlichen Niere mit einer Vielzahl von monoklonalen Antikörpern, von denen jeder verschieden ist und jeder von einer Hybridomzellinie produziert wird, die durch Fusion eines von einem Myelom stammenden Zellstammes und von Splenozyten, die von mit bekannten Kulturlinien aus der Gruppe menschliche Nierenepithelkrebszellen oder normale menschliche Nierenepithelzellen immunisierten BALB/c-Mäusen stammen, gebildet werden, unter geeigneten Bedingungen, so daß ein Komplex zwischen jedem Antikörper und einem Antigen gebildet wird, Nachweis der Gegenwart eines so gebildeten Komplexes, wobei die Abwesenheit eines Komplexes normale Nierenzellen anzeigt, und die Gegenwart eines Komplexes die Anwesenheit maligner menschlicher Nierenzellen anzeigt.

**10.** Verfahren nach Anspruch 9, wobei die Vielzahl der monoclonalen Antikörper ausgewählt ist aus der Gruppe, die aus $F_{23}$, $M_2$, $S_8$ und $S_{21}$ besteht.

**11.** Verfahren nach Anspruch 10, welches weiterhin das Inkontaktbringen einer Probe von einer menschlichen Niere mit einer Vielzahl von monoclonalen Antikörpern umfaßt, die ausgewählt sind aus der Gruppe, die aus $M_1$, $S_4$, $S_6$, $S_7$, $S_{22}$, $S_{23}$, $S_{24}$, $S_{25}$, $S_{26}$, $S_{27}$ und $V_1$ besteht.

**12.** Verfahren nach Anspruch 10, wobei das Verfahren als ein Test für das maligne Potential menschlicher Nierentumoren verwendet wird.

**13.** Antikörper-Antigen-Komplex, umfassend monoclonale Antikörper nach einem der Ansprüche 2 bis 4 und 8 und menschliche Nierenzellen.

**14.** Antikörper-Antigen-Komplex nach Anspruch 13, wobei die monoclonalen Antikörper an antigene Komponenten menschlicher Nierenzellen gebunden sind.